# DEMANDE DE BREVET EUROPEEN

(11) **EP 1 870 083 A2**
(43) Date de publication de la demande: **26.12.2007**
(21) Numéro de dépôt: 07109888.3
(22) Date de dépôt: 08.06.2007
(51) Int. Cl.: A61K 8/41, A61K 8/49, A61Q 5/10

(54) **Procédé pour la teinture prédictible des fibres kératiniques par application d'une composition contenant un dérivé diamino-N,N-dihydro-pyrazolone et d'une composition fondamentale ou a reflets dorés**

(30) Priorité: 20.06.2006 FR 0652549
(71) Demandeur: L'Oréal, 75008 Paris (FR)
(72) Inventeur: Cottard, François, 92400, Courbevoie (FR); Ascione, Jean-Marc, 75003, Paris (FR)
(74) Mandataire: Wattremez, Catherine

(57) **Abrégé**

L'invention a pour objet un procédé pour la coloration prédictible des fibres kératiniques, et en particulier des fibres kératiniques humaines telles que les cheveux, par application sur lesdites fibres d'une composition comprenant au moins une base d'oxydation dérivé de la diamino-N,N-dihydropyrazolone et au moins un coupleur particulier, mélangée à une composition « fondamentale » et/ou « fondamentale dorée » et/ou « dorée ».

La présente invention permet en particulier d'obtenir une coloration des fibres kératiniques, tenace, résistante à la lumière et au lavage.

## Description

L'invention a pour objet un procédé pour la coloration prédictible des fibres kératiniques, et en particulier des fibres kératiniques humaines telles que les cheveux, par application sur lesdites fibres d'une composition comprenant au moins une base d'oxydation dérivé de la diamino-N,N-dihydropyrazolone et au moins un coupleur particulier, mélangée à une composition « fondamentale » et/ou « fondamentale dorée » et/ou « dorée ».

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés d'indole, des dérivés d'indoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants. On obtient ainsi des colorations permanentes.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques.

La variété des molécules mises en jeu au niveau des bases d'oxydation et des coupleurs permet l'obtention d'une riche palette de couleurs.

L'utilisation de bases d'oxydation telle que les dérivés de paraphénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, de variété de nuances, d'uniformité de la couleur et de la ténacité aux agents extérieurs.

Dans les procédés de coloration d'oxydation traditionnelle, l'utilisateur professionnel ou non, souhaitant obtenir des couleurs prévisibles, peut mélanger des compositions de nuances différentes pour accéder à la couleur voire à la nuance intermédiaire désirée. Par exemple il est attendu qu'une composition tinctoriale « blond » mélangée à une composition « reflet cuivré » donne une coloration des fibres kératiniques, « blond à reflet cuivré ». L'intérêt de ces mélanges réside donc en la possibilité de pouvoir prédire la coloration nuancée, et ainsi à ne pas limiter la créativité de l'utilisateur.

Or certains précurseurs de coloration d'oxydation, ne permettent pas d'atteindre une coloration prédictible en mélange à une autre composition contenant des nuances « fondamentales » et/ou « fondamentales dorées » et/ou « dorées ». Le résultat du mélange des compositions tinctoriales est hasardeux ou fantaisiste. Notamment il s'est avéré que le résultat de la couleur sur les fibres kératiniques était peu prédictif lors de mélanges réalisés par le praticien coiffeur entre les nuances « reflet cuivré » ou « rouge clair » et des compositions « fondamentales » et/ou « des fondamentales dorées » et/ou « dorées », lorsqu'une des compositions contient notamment le coupleur 6-chloro-2-méthyl-5-aminophénol.

Aussi, le but de la présente invention est de fournir un nouveau procédé de coloration des fibres kératiniques qui permettent une coloration prédictible avec des nuances variées, puissantes, chromatiques, esthétiques, peu sélectives, et résistant bien aux diverses agressions que peuvent subir les cheveux tels que les shampooings, la lumière, la sueur et les déformations permanentes.

Ce problème technique a été résolu par un le procédé de coloration de fibres kératiniques consistant à appliquer sur les fibres un mélange d'une composition (A) et d'une composition tinctoriale (B) « fondamentale » ou « fondamentale dorée » ou « dorée »; ladite composition (A) comprenant au moins un coupleur 6-chloro-2-méthyl-5-aminophénol et au moins une base d'oxydation dérivée de la diamino-N,N-dihydro-pyrazolone de formule (I) ou l'un de ses sels, 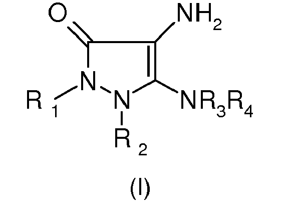 dans laquelle :
- R₁, R₂, R₃ et R₄, identiques ou différents, représentent :
   - un radical alkyle en C₁-C₆ linéaire ou ramifié éventuellement substitué par un ou plusieurs radicaux choisi dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un ou plusieurs groupes (C₁-C₄)alkyle, hydroxy, alcoxy en C₁-C₂, amino, (di)alkyl(C₁-C₂)amino ;
   - un radical aryle éventuellement substitué par un ou plusieurs (C₁-C₄)alkyle, hydroxy, alcoxy en C₁-C₂, amino, (di)alkyl(C₁-C₂)amino ;
   - un radical hétéroaryle à 5 ou 6 chaînons, éventuellement substitué par un ou plusieurs radicaux choisis parmi (C₁-C₄)alkyle, (C₁-C₂)alcoxy;
- R₃ et R₄ peuvent représenter également un atome d'hydrogène ;
- R₅, R₆ et R₇, identiques ou différents, représentent
   - un atome d'hydrogène ;
   - un radical alkyle linéaire ou ramifié en C₁-C₄ éventuellement substitué par un ou plusieurs radicaux choisis parmi hydroxy, alcoxy en C₁-C₂, carboxamido CONR₈R₉, sulfonyle SO₂R₈, aryle éventuellement substitué par un (C₁-C₄)alkyle, hydroxy, alcoxy en C₁-C₂, amino, (di)alkyl(C₁-C₂)amino ; aryle éventuellement substitué par un (C₁-C₄)alkyle , hydroxy, alcoxy en C₁-C₂, amino, (di)alkyl(C₁-C₂)amino ;
- R₆ et R₇, identiques ou différents, peuvent représenter également un radical carboxamido CONR₈R₉ ; un radical sulfonyle SO₂R₈ ;
- R₈ et R₉, identiques ou différents, représentent un atome d'hydrogène ; un radical alkyle en C₁-C₄ linéaire ou ramifié éventuellement substitués par un ou plusieurs hydroxy, alcoxy en C₁-C₂ ;
- R₁ et R₂ d'une part, et R₃ et R₄ d'autre part peuvent former avec le ou les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
- R₃ et R₄ peuvent également former ensemble avec l'atome d'azote auquel ils sont rattachés, un hétérocycle à 5 ou 7 chaînons dont les atomes de carbone peuvent être remplacés par un atome d'oxygène ou d'azote éventuellement substitué.

De façon inattendue lorsque le coupleur de type méta-aminophénol substitué tel que le 6-chloro-2-méthyl-5-aminophénol dans la composition (A), est associé à au moins une base d'oxydation de formule (I) telle que la 2,3-diamino-6,7-dihydro-1H,5H-pyrazolo[1,2-a]pyrazol-1-one dans la même composition (A), permet d'obtenir des colorations prédictibles sur la couleur des fibres kératiniques, une fois mélangé avec une composition tinctoriale (B) « fondamentale » et/ou « fondamentale dorée » et/ou « dorée ». La coloration obtenue après l'application du mélange sur lesdites fibres est celle attendue i.e. les fibres kératiniques sont colorées avec des nuances de reflets « cuivrés » ou « rouges ».

Cette coloration est par ailleurs puissante, esthétique, peu sélective et résistante aux diverses agressions que peuvent subir les cheveux tels que les shampooings, la lumière, la sueur et les déformations permanentes. Elle permet également d'obtenir des colorations intenses et variées à pH neutre. Elle permet notamment d'obtenir des nuances naturelles.

Dans le cadre de la présente invention un radical alkyle, correspond à une chaîne hydrocarbonée, saturée, linéaires ou ramifiés, de préférence en C₁-C₁₀ sauf indication contraire, préférentiellement en C₁-C₆, de préférence C₁-C₄ tels que le radical méthyle, éthyle, propyle, isopropyle, isobutyle, tertiobutyle, pentyle, hexyle.

Dans le cadre de la présente invention, le ou les hétéroatomes peuvent être choisis parmi un atome d'oxygène, un atome d'azote, un atome de soufre, un atome de phosphore.

Dans le cadre de la présente invention, un atome d'halogène peut être choisi parmi un atome de chlore, un atome de brome, un atome d'iode et un atome de fluor.

Plus particulièrement, dans la formule (I) les radicaux R₁ et R₂, identiques ou différents, sont choisis parmi
- un radical alkyle en C₁-C₆, de préférence C₁-C₄, éventuellement substitué par un hydroxy, un (C₁-C₂)alcoxy, un amino, un (di)alkyl(C₁-C₂)amino ;
- un radical phényle, méthoxyphényle, éthoxyphényle, benzyle.

De préférence, les radicaux R₁ et R₂, identiques ou non, sont choisis parmi un radical méthyle, éthyle, 2-hydroxyéthyle, 3-hydroxypropyle, 2-hydroxypropyle, phényle.

Selon un autre mode de réalisation, les radicaux R₁ et R₂ forment ensemble avec les atomes d'azotes auxquels ils sont rattachés, un cycle à 5 ou 6 chaînons, saturé ou insaturé, éventuellement substitué.

De préférence, les radicaux R₁ et R₂ forment ensemble avec les atomes d'azotes auxquels ils sont rattachés, un cycle pyrazolidine, pyridazolidine, éventuellement substitué par un ou plusieurs radicaux alkyle en C₁-C₄, hydroxy, (C₁-C₂)alcoxy, carboxy, carboxamido, amino, (di)alkyl(C₁-C₂)amino.

De manière encore plus avantageuse, les radicaux R₁ et R₂ forment ensemble avec les atomes d'azotes auxquels ils sont rattachés, un cycle pyrazolidine, pyridazolidine.

En ce qui concerne les radicaux R₃ et R₄, ces derniers, identiques ou différents, sont plus particulièrement choisis parmi un atome d'hydrogène ; un radical alkyle en C₁-C₆ de préférence C₁-C₄, linéaire ou ramifié, éventuellement substitué par un ou plusieurs hydroxy, (C₁-C₂)alcoxy, amino, un (di)alkyl(C₁-C₂)amino ; un radical phényle éventuellement substitué par un ou plusieurs radicaux hydroxy, amino, (C₁-C₂)alcoxy.

De préférence, les radicaux R₃ et R₄, identiques ou non, sont choisis parmi un atome d'hydrogène, un radical méthyle, éthyle, isopropyle, 2-hydroxyéthyle, 3-hydroxypropyle, 2-hydroxypropyle, 2-carboxyéthyle. Selon un mode de réalisation particulier, les radicaux R₃ et R₄, représentent un atome d'hydrogène.

Selon un autre mode de réalisation, les radicaux R₃ et R₄ forment ensemble avec l'atome d'azote auquel ils sont rattachés, un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl(C₁-C₂)amino, carboxy, carboxamido, alkyle en C₁-C₄ éventuellement substitué par un ou plusieurs radicaux hydroxy, amino, (di)alkylamino en C₁-C₂.

Plus particulièrement, les radicaux R₃ et R₄ forment ensemble avec l'atome d'azote auquel ils sont rattachés un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino- pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

De préférence, les radicaux R₃ et R₄ forment ensemble avec l'atome d'azote auquel ils sont rattachés un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthyl homopipérazine, la N β-hydroxyéthylhomopipérazine.

Conformément à un mode de réalisation encore plus préféré de l'invention, les radicaux R₃ et R₄ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

Les composés de formule (I) peuvent être éventuellement salifiés par des acides minéraux forts tels que par exemple HCl, HBr, Hl, H₂SO₄, H₃PO₄, ou des acides organiques tels que, par exemple, l'acide acétique, lactique, tartrique, citrique ou succinique, benzènesulfonique, para-toluènesulfonique, formique, ou C₁-C₄alkyl-SO₃H tel que l'acide méthanesulfonique.

Ils peuvent aussi être sous forme de solvates par exemple un hydrate ou un solvate d'alcool linéaire ou ramifié tel que l'éthanol ou l'isopropanol.

A titre d'exemples de dérivés de formule (I), on peut citer les composés présentés ci-dessous ou leurs sels d'addition :
4,5-diamino-1,2-diméthyl-1,2-dihydro-pyrazol-3-one;
4-amino-5-méthylamino-1 ,2-diméthyl-1 ,2-dihydro-pyrazol-3-one ;
4-amino-5-diméthylamino-1 ,2-diméthyl-1 ,2-dihydro-pyrazol-3-one ;
4-amino-5-(2-hydroxyéthyl)amino-1 ,2-diméthyl-1 ,2-dihydro-pyrazol-3-one ;
4-amino-5-(pyrrolidin-1-yl)-1,2-diméthyl-1,2-dihydro-pyrazol-3-one;
4-amino-5-(pipéridin-1-yl)-1,2-diméthyl-1,2-dihydro-pyrazol-3-one ;

4,5-diamino-1,2-di-(2-hydroxyéthyl)-1,2-dihydro-pyrazol-3-one;
4-amino-5-méthylamino-1,2-di-(2-hydroxyéthyl)-1,2-dihydro-pyrazol-3-one;
4-amino-5-diméthylamino-1,2-di-(2-hydroxyéthyl)-1,2-dihydro-pyrazol-3-one;
4-amino-5-(2-hydroxyéthyl)amino-1,2-di-(2-hydroxyéthyl)-1,2-dihydro-pyrazol-3-one;
4-amino-5-(pyrrolidin-1-yl)-1,2-di-(2-hydroxyéthyl)-1,2-dihydro-pyrazol-3-one;
4-amino-5-(pipéridin-1-yl)-1,2-di-(2-hydroxyéthyl)-1,2-dihydro-pyrazol-3-one;

4,5-diamino-1,2-diéthyl-1,2-dihydro-pyrazol-3-one;
4,5-diamino-1,2-phényl-1,2-dihydro-pyrazol-3-one;
4,5-diamino-1-éthyl-2-méthyl-1,2-dihydro-pyrazol-3-one;
4,5-diamino-2-éthyl-1 -méthyl-1,2-dihydro-pyrazol-3-one;
4,5-diamino-1-phényl-2-méthyl-1,2-dihydro-pyrazol-3-one;
4,5-diamino-2-phényl-1-méthyl-1,2-dihydro-pyrazol-3-one;
4,5-diamino-1-(2-hydroxyéthyl)-2-méthyl-1,2-dihydro-pyrazol-3-one;
4,5-diamino-2-(2-hydroxyéthyl)-1-méthyl-1,2-dihydro-pyrazol-3-one;

2,3-diamino-6,7-dihydro-1H,5H-pyrazolo[1,2-a]pyrazol-1-one ;
2-amino-3-méthylamino-6,7-dihydro-1H,5H-pyrazolo[1,2-a]pyrazol-1-one ;
2-amino-3-diméthylamino-6,7-dihydro-1 H,5H-pyrazolo[1,2-a]pyrazol-1-one ;
2-amino-3-éthylamino-6,7-dihydro-1H,5H-pyrazolo[1,2-a]pyrazol-1-one ;
2-amino-3-isopropylamino-6,7-dihydro-1H,5H-pyrazolo[1,2-a]pyrazol-1-one ;
2-amino-3-(2-hydroxyéthyl)amino-6,7-dihydro-1H,5H-pyrazolo[1,2-a]pyrazol-1-one ;
2-amino-3-(2-hydroxypropyl)amino-6,7-dihydro-1 H,5H-pyrazolo[1,2-a]pyrazol-1-one ;
2-amino-3-bis(2-hydroxyéthyl)amino-6,7-dihydro-1H,5H-pyrazolo[1,2-a]pyrazol-1-one ;

2-amino-3-(pyrrolidin-1-yl)-6,7-dihydro-1H,5H-pyrazolo[1,2-a]pyrazol-1-one;
2-amino-3-(3-hydroxy-pyrrolidin-1-yl)-6,7-dihydro-1H,5H-pyrazolo[1,2-a]pyrazol-1-one ;
2-amino-3-(pipéridin-1-yl)-6,7-dihydro-1H,5H-pyrazolo[1,2-a]pyrazol-1-one ;

2,3-diamino-6-hydroxy-6,7-dihydro-1H,5H-pyrazolo[1,2-a]pyrazol-1-one;
2,3-diamino-6-méthyl-6,7-dihydro-1H,5H-pyrazolo[1,2-a]pyrazol-1-one;
2,3-diamino-6-diméthyl-6,7-dihydro-1H,5H-pyrazolo[1,2-a]pyrazol-1-one;

2,3-diamino-5,6,7,8-tétrahydro-1H,6H-pyridazino[1,2-a]pyrazol-1-one;
2,3-diamino-5,8-dihydro-1H,6H-pyridazino[1,2-a]pyrazol-1-one;

4-Amino-5-diméthylamino-1,2-diethyl-1,2-dihydro-pyrazol-3-one;
4-Amino-1,2-diéthyl-5-ethylamino-1,2-dihydro-pyrazol-3-one;
4-Amino-1,2-diéthyl-5-isopropylamino-1,2-dihydro-pyrazol-3-one;
4-Amino-1,2-diéthyl-5-(2-hydroxy-ethylamino)-1,2-dihydro-pyrazol-3-one;
4-Amino-5-(2-diméthylamino-éthylamino)-1,2-diethyl-1,2-dihydro-pyrazol-3-one;
4-Amino-5-[bis-(2-hydroxy-éthyl)-amino]-1,2-diethyl-1,2-dihydro-pyrazol-3-one ;
4-Amino-1,2-diéthyl-5-(3-imidazol-1-yl-propylamino)-1,2-dihydro-pyrazol-3-one ;
4-Amino-5-diméthylamino-1,2-diéthyl-1,2-dihydro-pyrazol-3-one;
4-Amino-1,2-diéthyl-5-ethylamino-1,2-dihydro-pyrazol-3-one;
4-Amino-1,2-diéthyl-5-isopropylamino-1,2-dihydro-pyrazol-3-one;
4-Amino-1,2-diéthyl-5-(2-hydroxy-ethylamino)-1,2-dihydro-pyrazol-3-one;
4-Amino-5-(2-diméthylamino-éthylamino)-1,2-diéthyl-1,2-dihydro-pyrazol-3-one;
4-Amino-5-[bis-(2-hydroxy-éthyl)-amino]-1,2-diethyl-1,2-dihydro-pyrazol-3-one;
4-Amino-1,2-diéthyl-5-(3-imidazol-1-yl-propylamino)-1,2-dihydro-pyrazol-3-one;
4-Amino-1.2-diéthyl-5-(3-hydroxy-pyrrolidin-1-yl)-1,2-dihydro-pyrazol-3-one;
4-Amino-1.2-diéthyl-5-pyrrolidin-1-yl-1,2-dihydro-pyrazol-3-one;
4-Amino-5-(3-diméthylamino-pyrrolidin-1-yl)-1,2-diethyl-1,2-dihydro-pyrazol-3-one;
4-Amino-1,2-diethyl-5-(4-méthyl-pipérazin-1-yl)-pyrazolidin-3-one;
2,3-Diamino-6-hydroxy-6,7-dihydro-5H-pyrazolo[1,2-a]pyrazol-1-one;
dont certains sont figurés ci-dessous pour illustrer les noms par des structures chimiques :

| | |
|---|---|
| 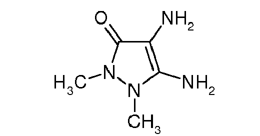 | 4,5-Diamino-1,2-diméthyl-1,2-dihydro-pyrazol-3-one |
| 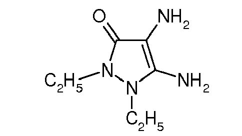 | 4,5-Diamino-1,2-diéthyl-1,2-dihydro-pyrazol-3-one; |
| 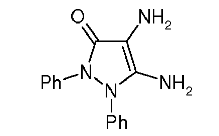 | 4,5-Diamino-1,2-diphényl-1,2-dihydro-pyrazol-3-one |
| 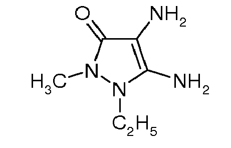 | 4,5-Diamino-1-éthyl-2-méthyl-1,2-dihydro-pyrazol-3-one ; |
| 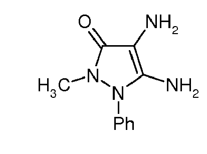 | 4,5-Diamino-1-phényl-2-méthyl-1,2-dihydro-pyrazol-3-one; |
| 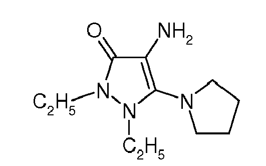 | 4-Amino-5-(pyrrolidin-1-yl)-1,2-diethyl-1,2-dihydro-pyrazol-3-one; |
| 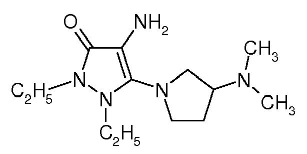 | 4-Amino-5-(3-diméthylamino-pyrrolidin-1-yl)-1,2-diethyl-1,2-dihydro-pyrazol-3-one; |
| 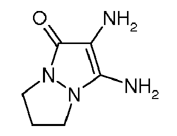 | 2,3-diamino-6,7-dihydro-1H,5H-pyrazolo[1,2-a]pyrazol-1-one; |
| 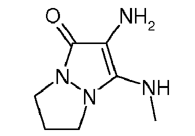 | 2-Amino-3-méthylamino-6,7-dihydro-1H,5H-pyrazolo[1,2-a]pyrazol-1-one; |
| 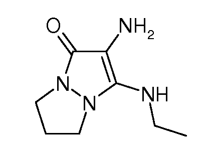 | 2-Amino-3-éthylamino-6,7-dihydro-1H,5H-pyrazolo[1,2-a]pyrazol-1-one ; |
| 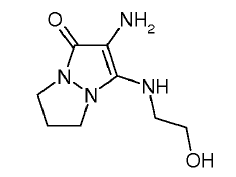 | 2-amino-3-(2-hydroxyéthyl)amino-6,7-dihydro-1H,5H-pyrazolo[1,2-a]pyrazol-1-one; |
| 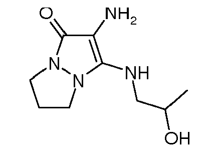 | 2-amino-3-(2-hydroxypropyl)amino-6,7-dihydro-1H,5H-pyrazolo[1,2-a]pyrazol-1-one; |
| 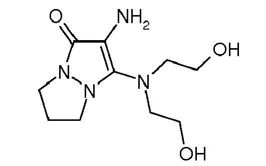 | 2-amino-3-bis(2-hydroxyéthyl)amino-6,7-dihydro-1H,5H-pyrazolo[1,2-a]pyrazol-1-one; |
| 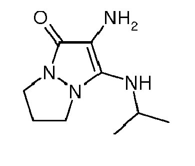 | 2-amino-3-isopropylamino-6,7-dihydro-1H,5H-pyrazolo[1,2-a]pyrazol-1-one ; |
| 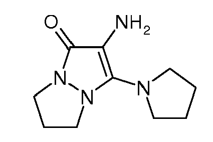 | 2-amino-3-(pyrrolidin-1-yl)-6,7-dihydro-1H,5H-pyrazolo[1,2-a]pyrazol-1-one; |
| 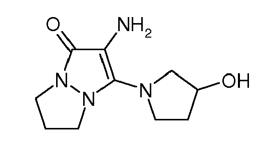 | 2-amino-3-(3-hydroxy-pyrrolidin-1-yl)-6,7-dihydro-1H,5H-pyrazolo[1,2-a]pyrazol-1-one ; |
| 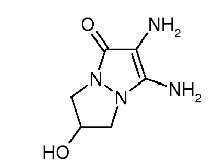 | 2,3-diamino-6-hydroxy-6,7-dihydro-1H,5H-pyrazolo[1,2-a]pyrazol-1-one; |
| 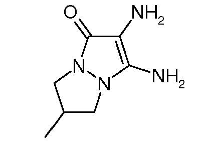 | 2,3-diamino-6-méthyl-6,7-dihydro-1H,5H-pyrazolo[1,2-a]pyrazol-1-one |
| 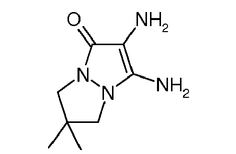 | 2,3-diamino-6,6-diméthyl-6,7-dihydro-1H,5H-pyrazolo[1,2-a]pyrazol-1-one; |
| 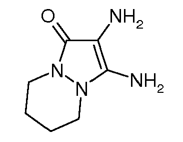 | 2,3-diamino-5,6,7,8-tétrahydro-1 H,6H-pyridazino[1,2-a]pyrazol-1-one ; |
| 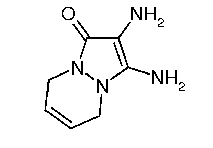 | 2,3-diamino-5,8-dihydro-1 H,6H-pyridazino[1,2-a]pyrazol-1-one ; |
| 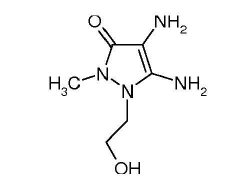 | 4,5-diamino-1-(2-hydroxyéthyl)-2-méthyl-1,2-dihydro-pyrazol-3-one ; |
| 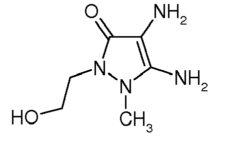 | 4,5-diamino-2-(2-hydroxyéthyl)-1-méthyl-1,2-dihydro-pyrazol-3-one. |

Parmi ces composés, les dérivés de diamino-*N*,*N*-dihydropyrazolone de formule (I) ou leurs sels d'addition, particulièrement préférés sont les :
2,3-Diamino-6,7-dihydro-1H,5H-pyrazolo[1,2-a]pyrazol-1-one;
2-Amino-3-éthylamino-6,7-dihydro-1H,5H-pyrazolo[1,2-a]pyrazol-1-one;
2-Amino-3-isopropylamino-6,7-dihydro-1H,5H-pyrazolo[1,2-a]pyrazol-1-one;
2-Amino-3-(pyrrolidin-1-yl)-6,7-dihydro-1H,5H-pyrazolo[1,2-a]pyrazol-1-one;
4,5-diamino-1,2-diméthyl-1,2-dihydro-pyrazol-3-one;
4,5-diamino-1,2-diéthyl-1,2-dihydro-pyrazol-3-one;
4,5-diamino-1,2-di-(2-hydroxyéthyl)-1,2-dihydro-pyrazol-3-one;
2-amino-3-(2-hydroxyéthyl)amino-6,7-dihydro-1H,5H-pyrazolo[1,2-a]pyrazol-1-one;
2-amino-3-diméthylamino-6,7-dihydro-1H,5H-pyrazolo[1,2-a]pyrazol-1-one;
2,3-diamino-5,6,7,8-tétrahydro-1H,6H-pyridazino[1,2-a]pyrazol-1-one;
4-Amino-1,2-diéthyl-5-(pyrrolidin-1-yl)-1,2-dihydro-pyrazol-3-one;
4-Amino-5-(3-diméthylamino-pyrrolidin-1-yl)-1,2-diéthyl-1,2-dihydro-pyrazol-3-one;
2,3-diamino-6-hydroxy-6,7-dihydro-1H,5H-pyrazolo[1,2-a]pyrazol-1-one.

On préfèrera encore plus particulièrement utiliser à titre de composé de formule (1) le 2,3-Diamino-6,7-dihydro-1H,5H-pyrazolo[1,2-a]pyrazol-1-one et ses sels d'addition.

La composition (A) contient au moins un coupleur de type méta-aminophénol substitué halogéné particulier choisi parmi le 6-chloro-2-méthyl-5-aminophénol et un de ses sels.

De préférence la quantité de 6-chloro-2-méthyl-5-aminophénol est comprise entre 0,001 et 8 % et plus préférentiellement entre 0,1 et 5 % du poids total de la composition tinctoriale (A).

La ou les bases d'oxydation de formule (1) sont en général présentes chacune en quantité comprise entre 0,001 à 8 % en poids environ du poids total de la composition tinctoriale (A), de préférence entre 0,1 et 5 %.

Un mode de réalisation particulier a pour objet une composition (A) de nuance cuivrée, dans laquelle le ratio molaire entre la base d'oxydation de formule (I) dont notamment la 2,3-diamino-6,7-dihydro-1H,5H-pyrazolo[1,2-a]pyrazol-1-one et le coupleur 6-chloro-2-méthyl-5-aminophénol, est inférieur à 1. Préférentiellement ledit ratio est compris entre 0,5 et 0,95.

Dans un autre mode de réalisation, la composition (A) de nuance cuivrée contient un autre coupleur préférentiellement méta-aminophénol substitué non halogéné, particulièrement le 2-méthyl-5-aminophénol, dont le ratio molaire entre la base d'oxydation de formule (I) notamment 2,3-diamino-6,7-dihydro-1H,5H-pyrazolo[1,2-a]pyrazol-1-one et le coupleur additionnel est supérieur à 1. Préférentiellement ledit ratio est compris entre 2 et 5.

Le composition (A) utile dans le procédé de l'invention peut contenir d'autres bases d'oxydation et d'autres coupleurs différents de ceux utiles dans la présente invention et conventionnellement utilisés pour la teinture de fibres kératiniques.

La composition (A) de la présente invention peut par exemple comprendre des bases d'oxydation additionnelles choisies parmi les para-phénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis précédemment et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènedlamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl)éthylènediamine, la N,N'-bis-(4-aminophényl)tétraméthylènediamine, la N,N'-bis-(β-hydroxyéthyl)N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino-1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino-3-méthyl 1-phényl pyrazole, le 4,5-diamino-1-méthyl 3-phényl pyrazole, le 4-amino-1,3-diméthyl 5-hydrazino pyrazole, le 1-benzyl-4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl-1-isopropyl pyrazole, le 4,5-diamino 3-méthyl-1-isopropyl pyrazole, le 4-amino-5-(2'-aminoéthyl)amino 1,3-diméthyl pyrazole, le 3,4,5-triamino pyrazole, le 1-méthyl-3,4,5-triamino pyrazole, le 3,5-diamino-1-méthyl 4-méthylamino pyrazole, le 3,5-diamino 4-(β-hydroxyéthyl)amino-1-méthyl pyrazole, et leurs sels d'addition avec un acide.

La ou les bases d'oxydation additionnelles présentes dans la composition (A) de l'invention sont en général présentes chacune en quantité comprise entre 0,001 à 10 % en poids environ du poids total de la composition tinctoriale, de préférence entre 0,005 et 6 %.

La composition tinctoriale (A) peut également comprendre des coupleurs additionnels autre que le 6-chloro-2-méthyl-5-aminophénol.

On peut notamment citer les méta-phénylènediamines, les méta-aminophénols, les méta-diphénols, les coupleurs naphtaléniques, les coupleurs hétérocycliques et leur sels d'addition.

Parmi les méta-aminophénols utiles dans le cadre de l'invention, on peut plus particulièrement citer le 3-aminophénol, le 5-amino-2-méthoxyphénol, le 5-amino 2-(β-hydroxyéthyloxy)phénol, le 5-amino-2-méthylphénol, le 5-N-(β-hydroxyéthyl)amino 2-méthylphénol, le 5-N-(β-hydroxyéthyl)amino 4-méthoxy 2-méthylphénol, le 5-amino-4-méthoxy-2-méthylphénol, le 5-amino 4-chloro-2-méthyl phénol, le 5-amino-2,4-diméthoxy phénol, le 5-(γ-hydroxypropylamino) 2-méthyl phénol, le 3-diméthylamino-phénol ; le 2-méthyl-5-diméthylamino-phénol ; le 2-éthyl-5-diméthylamino-phénol ; le 2-méthoxy-5-diméthylamino-phénol ; le 2-éthoxy-5-diméthylamino-phénol ; le 2-(β-hydroxyéthyl)-5-diméthylamino-phénol ; le 3-diéthylamino-phénol ; le 2-méthyl-5-diéthylamino-phénol ; le 2-éthyl-5-diéthylamino-phénol ; le 2-méthoxy-5-diéthylamino-phénol ; le 2-éthoxy-5-diéthylamino-phénol ; le 2-(β-hydroxyéthyl)-5-diéthylamino-phénol ; le 3-di(β-hydroxyéthyl)amino-phénol ; le 2-méthyl-5-di(β-hydroxyéthyl)aminophénol ; le 2-éthyl-5-di(β-hydroxyéthyl)amino-phénol ; le 2-méthoxy-5-di(β-hydroxyéthyl)amino-phénol ; le 2-éthoxy-5-di(β-hydroxyéthyl)amino-phénol ; le 2-(β-hydroxyéthyl)-5-di(β-hydroxyéthyl)amino-phénol ; le 3-pyrrolidin-1-yl-phénol ; le 2-méthyl-5-pyrrolidin-1-yl-phénol ; le 2-éthyl-5-pyrrolidin-1-yl-phénol ; le 2-méthoxy-5-pyrrolidin-1-yl-phénol ; le 2-éthoxy-5-pyrrolidin-1-yl-phénol ; le 2-(β-hydroxyéthyl)-5-pyrrolidin-1-yl-phénol ; le 3-pipéridin-1-yl-phénol ; le 2-méthyl-5-pipéridin-1-yl-phénol ; le 2-éthyl-5-pipéridin-1-yl-phénol ; le 2-méthoxy-5-pipéridin-1-yl-phénol ; le 2-éthoxy-5-pipéridin-1-yl-phénol ; le 2-(β-hydroxyéthyl)-5-pipéridin-1-yl-phénol ; le 3-pipérazin-1-yl-phénol ; le 2-méthyl-5-pipérazin-1-yl-phénol ; le 2-éthyl-5-pipérazin-1-yl-phénol ; le 2-méthoxy-5-pipérazin-1-yl-phénol ; le 2-éthoxy-5-pipérazin-1-yl-phénol ; le 2-(β-hydroxyéthyl)-5-pipérazin-1-yl-phénol ; le 3-(4-méthyl-pipérazin-1-yl)-phénol ; le 2-méthyl-5-(4-méthyl-pipérazin-1-yl)-phénol ; le 2-éthyl-5-(4-méthyl-pipérazin-1-yl)-phénol ; le 2-méthoxy-5-(4-méthyl-pipérazin-1-yl)-phénol ; le 2-éthoxy-5-(4-méthyl-pipérazin-1-yl)-phénol ; le 2-(β-hydroxyéthyl)-5-(4-méthyl-pipérazin-1-yl)-phénol ; le 3-(4-éthyl-pipérazin-1-yl)-phénol ; le 2-méthyl-5-(4-éthyl-pipérazin-1-yl)-phénol ; le 2-éthyl-5-(4-éthyl-pipérazin-1-yl)-phénol ; le 2-méthoxy-5-(4-éthyl-pipérazin-1-yl)-phénol ; le 2-éthoxy-5-(4-éthyl-pipérazin-1-yl)-phénol ; le 2-(β-hydroxyéthyl)-5-(4-éthyl-pipérazin-1-yl)-phénol ; le 3-(4-(β-hydroxyéthyl)-pipérazin-1-yl)-phénol ; le 2-méthyl-5-(4-(β-hydroxyéthyl)-pipérazin-1-yl)-phénol ; le 2-éthyl-5-(4-(β-hydroxyéthyl)-pipérazin-1-yl)-phénol ; le 2-méthoxy-5-(4-(β-hydroxyéthyl)-pipérazin-1-yl)-phénol ; le 2-éthoxy-5-(4-(β-hydroxyéthyl)-pipérazin-1-yl)-phénol ; le 2-(β-hydroxyéthyl)-5-(4-(β-hydroxyéthyl)-pipérazin-1-yl)-phénol ; le 3-morpholin-4-yl-phénol ; le 2-méthyl-5-morpholin-4-yl-phénol ; le 2-éthyl-5-morpholin-4-yl-phénol ; le 2-méthoxy-5-morpholin-4-yl-phénol ; le 2-éthoxy-5-morpholin-4-yl-phénol ; le 2-(β-hydroxyéthyl)-5-morpholin-4-yl-phénol, et leurs sels d'addition.

A titre d'exemple de coupleurs autres que les métaaminophénols, on peut citer le 1,3-dihydroxybenzène, le 1,3-dihydroxy-2-méthylbenzène, le 4-chloro-1,3-dihydroxybenzène, le 2,4-diamino-1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxybenzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)-toluène et leurs sels d'addition avec un acide.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, alkyltosylates, les phosphates et les acétates et les sels d'addition avec une base telles que la soude, la potasse, l'ammoniaque, les amines ou les alcanolamines.

La composition tinctoriale (A) conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

On entend par composition tinctoriale « fondamentale » ou « fondamentale dorée » ou « dorée » toute composition comprenant au moins une base d'oxydation et éventuellement au moins un coupleur, conduisant sur les fibres kératiniques à une nuance « fondamentale » ou « fondamentale dorée » ou « dorée » i.e. une nuance pouvant par exemple être définie par la formule générale ci-dessous :

**H,(O)**ₙ**(R**₁**)**ₙ₁**(R**₂**)**ₙ₂

dans laquelle :
- H représente la hauteur de ton de la nuance telle que définie dans l'ouvrage The Science of Hair Care, 2d Ed., Ed. Taylor and Francis Group, 2005, pp. 295 et H peut être comprise selon l'ouvrage inclusivement entre 1 et 10 ; elle est comprise selon l'invention entre 4 et 9 ; de préférence **H** est comprise entre 6 et 9 et plus préférentiellement entre 7 et 9 ;
- **R**₁ représente le reflet primaire ou dominant avec **R**₁=1 correspond au reflet cendré, **R**₁=2: reflet irisé, **R**₁=3: reflet doré, **R**₁=4: reflet cuivré, **R**₁=5: reflet acajou, **R**₁=6 reflet rouge, **R**₁=7 reflet vert ; **R**₁ selon l'invention est égal à 3;
- **R₂** représente le reflet secondaire ou correcteur;
- **n** représente un entier 0 ou 1 ; lorsque **n** représente 1, le signe **0** atténue la force des reflets **R**₁ (et **R**₂);
- **n**₁ représente un entier 0 ou 1 ;
- **n**₂ représente un entier 0 ou 1 ; lorsque **n**₂ représente 1, **R**₂ peut prendre chacune des valeurs de 1 à 7 mentionnées ci-dessus.

A titre d'exemple de nuances fondamentales ou fondamentales dorées ou dorées selon l'invention on peut citer les nuances 7 - 8 - 9 - 7,03 - 8,03 - 9,03 - 7,3 - 8,3 - 9,3.

De préférence **n₁** vaut 1. Plus préférentiellement **n₁** vaut **1** et **n** est nul.

Plus particulièrement, la composition « fondamentale » ou « fondamentale dorée » ou « dorée » est choisie de sorte qu'en présence d'un agent oxydant elle conduit à température ambiante sur une mèche de cheveux gris à 90 % blancs, non permanenté aux valeurs suivantes dans le système colorimétrique L*a*b*:
a* appartient à l'intervalle [0 ; +20], b* appartient à l'intervalle [0 ; +20], L* appartient à l'intervalle [0 ; +50].

Un mode de réalisation particulier de l'invention concerne, la composition (B) « fondamentale » dont les valeurs de L*a*b* sont les suivantes a* appartient à l'intervalle [0 ; +6], b* appartient à l'intervalle [+10 ; +15], L* appartient à l'intervalle [+20 ; +50].

Un autre mode de réalisation de l'invention concerne la composition (B) « fondamentale dorée» dont les valeurs de L*a*b*: sont les suivantes :
a* appartient à l'intervalle [+6 ; +10], et b* appartient à l'intervalle [+15 ; +20], L* appartient à l'intervalle [+20 ; +50].

La ou les bases d'oxydation contenues dans la composition (B) et la ou les coupleurs éventuellement contenus dans la composition (B), sont tels que définis précédemment pour les bases et coupleurs additionnels de la composition (A).

De préférence la composition (B) ne contient pas de composés de formule (I) ni de 6-chloro-2-méthyl-5-aminophénol.

Dans un mode de réalisation particulier de l'invention la composition (B) contient au moins une base d'oxydation choisie parmi les para-phénylènediamines dont notamment la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées. Préférentiellement la base d'oxydation de la composition (B) est choisie parmi la paratoluènediamine, la paraphénylènediamine et un de leurs sels.

Par ailleurs, dans un mode de réalisation avantageux la proportion pondérale de la ou des bases d'oxydation de la composition (B) est de préférence inférieure ou égale à 5 mmol pour 100 g de ladite composition.

Un autre mode de réalisation de l'invention concerne le ou les coupleurs de la composition (B) choisis parmi les méta-diphénols tels que le 1,3-dihydroxybenzène ; les méta-aminophénols tels que le 3-aminophénol, et les métaphénylènediamines telles que le 2,4-diamino 1-(β-hydroxyéthyloxy) benzène.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

Les compositions tinctoriales (A) et/ou (B), conformes à l'invention peuvent également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids des compositions tinctoriale (A) et (B).

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH des compositions tinctoriales (A) et/ou (B), est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 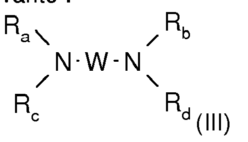 dans laquelle W est un reste propylène éventuellement substitué par un groupement hydroxyle ou un radical alkyle en C₁-C₄ Rₐ, R_{b}, R_{c} et R_{d}, identiques ou différents, représentent un atome d'hydrogène, un radical alkyle en C₁-C₄ ou hydroxyalkyle en C₁-C₄.

Les compositions tinctoriales (A) et/ou (B), peuvent se présenter sous des formes diverses, telles que sous forme de liquides, de crèmes, de gels, ou sous toute autre forme appropriée pour réaliser une teinture des fibres kératiniques, et notamment des cheveux humains.

Le mélange des deux compositions (A) et (B) se trouve de préférence dans un rapport (A)/(B) pondéral compris entre 5/1 et 1/5 et de préférence entre 3/1 et 1/3. Plus préférentiellement le rapport pondéral du mélange (A)/(B) est de 1/1.

Le mélange peut être préparé avant ou au moment de l'application sur les fibres kératiniques.

Le procédé de la présente invention est un procédé de coloration de fibres kératiniques consistant à appliquer sur les fibres un mélange d'une composition (A) et d'une composition tinctoriale (B) « fondamentale » ou « fondamentale dorée »;
ladite composition (A) comprenant au moins un coupleur choisi parmi le 6-chloro-2-méthyl-5-aminophénol et l'un de ses sels, et comprenant au moins une base d'oxydation dérivée de la diamino-N,N-dihydro-pyrazolone de formule (I) ou l'un de ses sels, 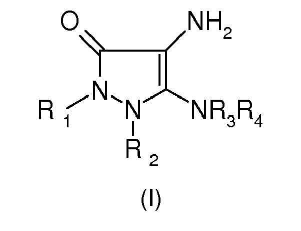 dans laquelle :
- R₁, R₂, R₃ et R₄, identiques ou différents, représentent :
   - un radical alkyle en C₁-C₆ linéaire ou ramifié éventuellement substitué par un ou plusieurs radicaux choisi dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un ou plusieurs groupes (C₁-C₄)alkyle, hydroxy, alcoxy en C₁-C₂, amino, (di)alkyl(C₁-C₂)amino;
   - un radical aryle éventuellement substitué par un ou plusieurs (C₁-C₄)alkyle, hydroxy, alcoxy en C₁-C₂, amino, (di)alkyl(C₁-C₂)amino ;
   - un radical hétéroaryle à 5 ou 6 chaînons, éventuellement substitué par un ou plusieurs radicaux choisis parmi (C₁-C₄)alkyle, (C₁-C₂)alcoxy ;
- R₃ et R₄ peuvent représenter également un atome d'hydrogène ;
- R₅, R₆ et R₇, identiques ou différents, représentent
   - un atome d'hydrogène ;
   - un radical alkyle linéaire ou ramifié en C₁-C₄ éventuellement substitué par un ou plusieurs radicaux choisis parmi hydroxy, alcoxy en C₁-C₂, carboxamido CONR₈R₉, sulfonyle SO₂R₈, aryle éventuellement substitué par un (C₁-C₄)alkyle, hydroxy, alcoxy en C₁-C₂, amino, (di)alkyl(C₁-C₂)amino ; aryle éventuellement substitué par un (C₁-C₄)alkyle , hydroxy, alcoxy en C₁-C₂, amino, (di)alkyl(C₁-C₂)amino ;
- R₆ et R₇, identiques ou différents, peuvent représenter également un radical carboxamido CONR₈R₉ ; un radical sulfonyle SO₂R₈ ;
- R₈ et R₉, identiques ou différents, représentent un atome d'hydrogène ; un radical alkyle en C₁-C₄ linéaire ou ramifié éventuellement substitués par un ou plusieurs hydroxy, alcoxy en C₁-C₂ ;
- R₁ et R₂ d'une part, et R₃ et R₄ d'autre part peuvent former avec le ou les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
- R₃ et R₄ peuvent également former ensemble avec l'atome d'azote auquel ils sont rattachés, un hétérocycle à 5 ou 7 chaînons dont les atomes de carbone peuvent être remplacés par un atome d'oxygène ou d'azote éventuellement substitué.

Selon un mode de réalisation particulier, le mélange de la composition (A) et (B) est ajouté de préférence au moment de l'emploi, à une composition contenant dans un milieu approprié pour la teinture, au moins un agent oxydant. Cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, plus préférentiellement 20 minutes, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement au mélange de compositions de l'invention.

De préférence, cette coloration est révélée à pH neutre.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme une composition tinctoriale (A) comprenant au moins un coupleur choisi parmi le 6-chloro-2-méthyl-5-aminophénol et un de ses sels, comprenant également au moins une base d'oxydation diamino-*N,N*-dihydro-pyrazolone de formule (1) telle que la 2,3-diamino-6,7-dihydro-1H,5H-pyrazolo[1,2-a]pyrazol-1-one; un deuxième compartiment renferme une composition (B) de nuance fondamentale ou fondamentale dorée et un troisième compartiment renferme l'agent oxydant ou une composition oxydante.

Ce dispositif peut être équipé d'un moyen permettant de délivrer sur les cheveux le mélange souhaité, tel que les dispositifs décrits dans le brevet FR-2 586 913 au nom de la demanderesse.

Un mode de réalisation particulier concerne des composés de formule un « kit » de teinture tel que défini précédemment pour lequel le premier compartiment contient une composition tinctoriale (A) de nuance cuivré, le deuxième compartiment contient un composition (B) de nuance fondamentale, et le dernier compartiment contient un agent oxydant.

Les exemples qui suivent servent à illustrer l'invention sans toutefois présenter un caractère limitatif.

### EXEMPLE DE PROCEDE DE COLORATION

### Composition (A) de la nuance « cuivrée » :

| | |
|---|---|
| ACIDE LAURIQUE NATUREL | 2 |
| ALCOOL DECYLIQUE OXYETHYLENE (3 OE) | 12 |
| ALCOOL LAURIQUE OXYETHYLENE (1 2 OE) | 6 |
| ALCOOL CETYLSTEARILYQUE (C16-C18 - 50/50) | 10 |
| ALCOOL OLEOCETYLIQUE OXYETHYLENE (30 OE) | 3.5 |
| SILICE PYROGENEE A CARACTERE HYDROPHOBE | 1 |
| MONOETHANOLAMINE PURE | 1.2 |
| POLYQUATERNIUM 6 | 5 |
| PROPYLENE GLYCOL | 7 |
| HEXADIMETHRINE CHLORIDE | 3 |
| ACIDE POLYACRYLIQUE RETICULE | 0.6 |
| ACIDE DIETHYLENE TRIAMINE PENTACETIQUE, SEL PENTASODIQUE EN SOLUTION AQUEUSE A 40 % | 2 |
| THIOLACTATE D'AMMONIUM EN SOLUTION AQUEUSE A 58 % (50 % EN ACIDE THIOLACTIQUE) | 0.8 |
| META BISULFITE DE SODIUM | 0.71 |
| ACIDE ASCORBIQUE | 0.25 |
| PARA-AMINOPHENOL | 0.1 |
| 6-CHLORO-2-METHYL-5-AMINOPHENOL | 0.8 |
| 2,3-DIAMINO-6,7-DIHYDRO-1H,5H-PYRAZOLO[1,2- A]PYRAZOL-1-ONE DIMETHANESULFONATE | 1.9 |
| 1-METHYL-2-HYDROXY-4-AMINO-BENZENE | 0.2 |
| AMMONIAQUE (A 20.5 % EN AMMONIAC) | 12 |
| PARFUM | 0.5 |
| EAU DESIONISEE (QS) | Qsp 100 |

### Composition (B) de la nuance « dorée » :

| | |
|---|---|
| ACIDE LAURIQUE NATUREL | 2 |
| ALCOOL DECYLIQUE OXYETHYLENE (3 OE) | 12 |
| ALCOOL LAURIQUE OXYETHYLENE (1 2 OE) | 6 |
| ALCOOL CETYLSTEARILYQUE (C16-C18 - 50/50) | 10 |
| ALCOOL OLEOCETYLIQUE OXYETHYLENE (30 OE) | 3.5 |
| SILICE PYROGENEE A CARACTERE HYDROPHOBE | 1 |
| MONOETHANOLAMINE PURE | 1.2 |
| POLYQUATERNIUM 6 | 5 |
| PROPYLENE GLYCOL | 7 |
| HEXADIMETHRINE CHLORIDE | 3 |
| ACIDE POLYACRYLIQUE RETICULE | 0.6 |
| ACIDE DIETHYLENE TRIAMINE PENTACETIQUE, SEL PENTASODIQUE EN SOLUTION AQUEUSE A 40 % | 2 |
| THIOLACTATE D'AMMONIUM EN SOLUTION AQUEUSE A 58 % (50 % EN ACIDE THIOLACTIQUE) | 0.8 |
| META BISULFITE DE SODIUM | 0.71 |
| ACIDE ASCORBIQUE | 0.25 |
| 1-METHYL-2,5-DIAMINOBENZENE | 0.33 |
| PARAAMINOPHENOL | 0.55 |
| RESORCINOL | 0.64 |
| META AMINOPHENOL | 0.07 |
| 6-HYDROXYINDOLE | 0.055 |
| AMMONIAQUE (A 20.5 % EN AMMONIAC) | 12 |
| PARFUM | 0.5 |
| EAU DESIONISEE (QS) | Qsp 100 |

### Composition (C) de la « nuance cuivrée » dont la base d'oxydation dérivée de diamino-N,N-dihydropyrazolone a été remplacée par la para-aminophénol, et contenant le coupleur spécifique 6-chloro-2-méthyl-5-aminophénol

| | |
|---|---|
| ACIDE LAURIQUE NATUREL | 2 |
| ALCOOL DECYLIQUE OXYETHYLENE (3 OE) | 12 |
| ALCOOL LAURIQUE OXYETHYLENE (1 2 OE) | 6 |
| ALCOOL CETYLSTEARILYQUE (C16-C18 - 50/50) | 10 |
| ALCOOL OLEOCETYLIQUE OXYETHYLENE (30 OE) | 3.5 |
| SILICE PYROGENEE A CARACTERE HYDROPHOBE | 1 |
| MONOETHANOLAMINE PURE | 1.2 |
| POLYQUATERNIUM 6 | 5 |
| PROPYLENE GLYCOL | 7 |
| HEXADIMETHRINE CHLORIDE | 3 |
| ACIDE POLYACRYLIQUE RETICULE | 0.6 |
| ACIDE DIETHYLENE TRIAMINE PENTACETIQUE, SEL PENTASODIQUE EN SOLUTION AQUEUSE A 40 % | 2 |
| THIOLACTATE D'AMMONIUM EN SOLUTION AQUEUSE A 58 % (50 % EN ACIDE THIOLACTIQUE) | 0.8 |
| META BISULFITE DE SODIUM | 0.71 |
| ACIDE ASCORBIQUE | 0.25 |
| PARAAMINOPHENOL | 0.6 |
| RESORCINOL | 0.01 |
| 6-CHLORO-2-METHYL-5-AMINOPHENOL | 1.4 |
| 6-HYDROXYINDOLE | 0.05 |
| AMMONIAQUE (A 20.5 % EN AMMONIAC) | 12 |
| PARFUM | 0.5 |
| EAU DESIONISEE (QS) | Qsp 100 |

### Mode d'application et résultats

Au moment de l'emploi, la composition (A) ou (C) est mélangée à la composition (B) dans un rapport pondéral de 1/1 puis le mélange résultant est mélangé avec un agent oxydant dans les proportions pondérales de 1/1,5 (1+1,5). L'agent oxydant est un agent oxydant de type peroxyde d'hydrogène à 25 volumes (25 V). On obtient un pH voisin de 10.

Les mélanges (A)/(B) ou (C)/(B), sont appliqués sur des mèches de cheveux gris à 90 % de blancs naturels (BN) à raison de 30 g de mélange pour 2 g de cheveux. Après 20 minutes de pose à température ambiante, les mèches sont rincées, lavées avec un shampooing standard, rincées à nouveau puis séchées.

| Tableau de résultats : | Couleur des fibres kératiniques 90% BN | **H** Hauteur de ton | Reflets des fibres kératiniques 90% BN | **(R₁)(R₂)** |
|---|---|---|---|---|
| Composition (A) + oxydant* | Blond cuivré | 7 | Cuivré | 4 |
| Composition (B) + oxydant* | Blond doré | 7 | Doré | 3 |
| Composition (C) + oxydant* | Blond cuivré | 7 | Cuivré | 4 |
| Mélange (A)/(B) + oxydant* | Blond cuivré doré | 7 | Cuivré | 4 3 |
| Mélange (C)/(B) + oxydant* | Blond violacé | 7 | Irisé doré | 2 3 |

| | | | | |
|---|---|---|---|---|
| Oxydant* : peroxyde d'hydrogène à 25 Volumes | | | | |

Ainsi, de façon prévisible le mélange des compositions (A)/(B) colore les fibres kératiniques avec une nuance cuivrée qui couvre parfaitement les cheveux blancs.

En revanche, le mélange des compositions (C)/(B) colore les fibres en blond avec des nuances indésirables « violacées ».

Il apparaît donc que la présence de la base d'oxydation de type diamino-N,N-dihydropyrazolone de formule (1) tel que le sel de 2,3-diamino-6,7-dihydro-1 H,5H-pyrazolo[1,2-a]pyrazol-1-one dans la composition (A), permet d'éviter une couleur et/ou une nuance non prédictible provenant du mélange de « fondamentale » ou de « fondamentale dorée » avec une composition tinctoriale « nuancée» contenant un coupleur méta-aminophénols substitué par un halogène, dont notamment le 6-chloro-2-méthyl-5-aminophénol.

## Revendications

1. Procédé de coloration de fibres kératiniques consistant à appliquer sur les fibres un mélange d'une composition (A) et d'une composition tinctoriale (B) « fondamentale » ou « fondamentale dorée » ou « dorée »;
ladite composition (A) comprenant au moins un coupleur choisi parmi le 6-chloro-2-méthyl-5-aminophénol et l'un de ses sels, et comprenant au moins une base d'oxydation dérivée de la diamino-N,N-dihydro-pyrazolone de formule (1) ou l'un de ses sels, 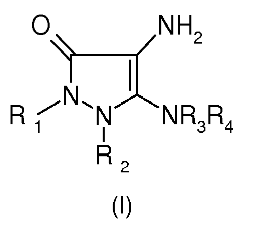 dans laquelle :
• R₁, R₂, R₃ et R₄, identiques ou différents, représentent :
- un radical alkyle en C₁-C₆ linéaire ou ramifié éventuellement substitué par un ou plusieurs radicaux choisi dans le groupe constitué par un radical OR₅, un radical NR₆R₇, un radical carboxy, un radical sulfonique, un radical carboxamido CONR₆R₇, un radical sulfonamido SO₂NR₆R₇, un hétéroaryle, un aryle éventuellement substitué par un ou plusieurs groupes (C₁-C₄)alkyle, hydroxy, alcoxy en C₁-C₂, amino, (di)alkyl(C₁-C₂)amino ;
- un radical aryle éventuellement substitué par un ou plusieurs (C₁-C₄)alkyle, hydroxy, alcoxy en C₁-C₂, amino, (di)alkyl(C₁-C₂)amino ;
- un radical hétéroaryle à 5 ou 6 chaînons, éventuellement substitué par un ou plusieurs radicaux choisis parmi (C₁-C₄)alkyle, (C₁-C₂)alcoxy ;
• R₃ et R₄ peuvent représenter également un atome d'hydrogène ;
• R₅, R₆ et R₇, identiques ou différents, représentent
- un atome d'hydrogène ;
- un radical alkyle linéaire ou ramifié en C₁-C₄ éventuellement substitué par un ou plusieurs radicaux choisis parmi hydroxy, alcoxy en C₁-C₂, carboxamido CONR₈R₉, sulfonyle SO₂R₈, aryle éventuellement substitué par un (C₁-C₄)alkyle, hydroxy, alcoxy en C₁-C₂, amino, (di)alkyl(C₁-C₂)amino ; aryle éventuellement substitué par un (C₁-C₄)alkyle , hydroxy, alcoxy en C₁-C₂, amino, (di)alkyl(C₁-C₂)amino ;
• R₆ et R₇, identiques ou différents, peuvent représenter également un radical carboxamido CONR₈R₉ ; un radical sulfonyle SO₂R₈ ;
• R₈ et R₉, identiques ou différents, représentent un atome d'hydrogène ; un radical alkyle en C₁-C₄ linéaire ou ramifié éventuellement substitués par un ou plusieurs hydroxy, alcoxy en C₁-C₂ ;
• R₁ et R₂ d'une part, et R₃ et R₄ d'autre part peuvent former avec le ou les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl(C₁-C₄)amino, hydroxy, carboxy, carboxamido, (C₁-C₂)alcoxy, les radicaux alkyles en C₁-C₄ éventuellement substitués par un ou plusieurs radicaux hydroxy, amino, (di)-alkylamino, alcoxy, carboxy, sulfonyle ;
• R₃ et R₄ peuvent également former ensemble avec l'atome d'azote auquel ils sont rattachés, un hétérocycle à 5 ou 7 chaînons dont les atomes de carbone peuvent être remplacés par un atome d'oxygène ou d'azote éventuellement substitué ;
en présence d'un agent oxydant.

2. Procédé de coloration selon la revendication précédentes dans lequel la base d'oxydation de la composition (A) de formule (1) comporte des groupements R₁ et R₂ qui forment ensemble avec les atomes d'azotes auxquels ils sont rattachés un cycle pyrazolidine, pyridazolidine, éventuellement substitué par un ou plusieurs radicaux alkyle en C₁-C₄, un hydroxy, un (C₁-C₂)alcoxy, un carboxy, un carboxamido, un amino, un (di)alkyl(C₁-C₂)amino.

3. Procédé de coloration selon une quelconque des revendications précédentes dans lequel la base d'oxydation de la composition (A) de formule (I) comporte des groupements R₃ et R₄ qui sont choisis parmi un atome d'hydrogène ; un radical alkyle en C₁-C₆ linéaire ou ramifié éventuellement substitué par un ou plusieurs hydroxy, (C₁-C₂)alcoxy, amino, un (di)alkyl(C₁-C₂)amino ; un radical phényle éventuellement substitué par un ou plusieurs radicaux hydroxy, amino, (C₁-C₂)alcoxy.

4. Procédé de coloration selon une quelconque des revendications précédentes dans lequel la base d'oxydation de la composition (A) est choisi parmi le 2,3-diamino-6,7-dihydro-1H,5H-pyrazolo[1,2-a]pyrazol-1-one et l'un de ses sels.

5. Procédé selon une quelconques des revendications précédentes dans lequel la quantité pondérale du coupleur 6-chloro-2-méthyl-5-aminophénol ou un de ses sels est comprise entre 0,001 et 8 %.

6. Procédé une quelconques des revendications précédentes dans lequel la quantité pondérale de base d'oxydation de formule (I) de la composition (A) est comprise entre 0,001 et 8 %.

7. Procédé de coloration selon une quelconque des revendications précédentes dans lequel le ratio pondéral du mélange de la composition (A) et de la composition (B) est un rapport (A)/(B) compris entre 5/1 et 1/5.

8. Procédé de coloration selon une quelconque des revendications précédentes dans lequel la composition (B) est une composition « fondamentale » ou « fondamentale dorée » ou « dorée » choisie de sorte qu'en présence d'un agent oxydant elle conduit à température ambiante sur une mèche de cheveux gris à 90 % blancs non permanentés, aux valeurs suivantes dans le système colorimétrique L*a*b*: a* appartient à l'intervalle [0 ; +20], b* appartient à l'intervalle [0 ; +20], L* appartient à l'intervalle [0 ; +50].

9. Procédé de coloration selon la revendication précédente dans lequel la composition (B) contient au moins une base d'oxydation choisi parmi les para-phénylènediamines et leurs sels.

10. Procédé de coloration selon la revendication précédente dans lequel les paraphénylènes diamines et leurs sels sont choisis parmi la paratoluènediamine, la paraphénylènediamine et leurs sels.

11. Procédé de coloration selon une quelconque des revendications 8 à 10, dans lequel la base d'oxydation de la composition (B) se trouve comprise à un taux inférieur ou égal à 5 mmol pour 100 g de composition (B).

12. Procédé de coloration selon une quelconque des revendications précédentes 8 à 11, dans lequel la composition (B) contient au moins un coupleur choisi parmi les méta-diphénols, les méta-aminophénols et les métaphénylènediamines.

13. Dispositif à plusieurs compartiments ou "kit" de teinture dans lequel un premier compartiment renferme une composition tinctoriale (A) comprenant au moins une base d'oxydation diamino-*N*,*N*-dihydro-pyrazolone de formule (I) telle que définie dans les revendications 1 à 4 et au moins un coupleur particulier choisi parmi le 6-chloro-2-méthyl-5-aminophénol et l'un de ses sels ; un deuxième compartiment renferme une composition (B) de nuance « fondamentale » ou « fondamentale dorée » ou « dorée » éventuellement dans des proportions pondérales telles que définie à la revendication 5, et un troisième compartiment renferme l'agent oxydant ou une composition oxydante.
